Europäisches Patentamt

European Patent Office

Office européen des brevets

(11) Publication number: **0 356 180**
**A1**

# (12) EUROPEAN PATENT APPLICATION

(21) Application number: **89308435.0**

(22) Date of filing: **21.08.89**

(51) Int. Cl.⁵: **A 61 K 37/02**

(30) Priority: **26.08.88 US 237142**

(43) Date of publication of application:
**28.02.90 Bulletin 90/09**

(84) Designated Contracting States: **ES GR**

(71) Applicant: **SCHERING CORPORATION**
**2000 Galloping Hill Road**
**Kenilworth New Jersey 07033 (US)**

(72) Inventor: **Chiu, Choy-Pik**
**10230 Yoshino Place**
**Cupertino California 95014 (US)**

**Lee, Frank D.**
**212 Rinconada Avenue**
**Palo Alto California 94301 (US)**

**Wideman, Janusz W.**
**3406 Thomas Drive**
**Palo Alto California 94303 (US)**

(74) Representative: **Ritter, Stephen David et al**
**Mathys & Squire 10 Fleet Street**
**London EC4Y 1AY (GB)**

(54) Treatment of myeloid leukemias by inducing terminal differentiation with interleukin-6.

�७ A method of treating myeloid leukemias comprises administering to a patient an effective amount of human interleukin-6 for inducing immature leukemic myeloid cells to terminally differentiate into functional adult cells.

EP 0 356 180 A1

Description

## TREATMENT OF MYELOID LEUKEMIAS BY INDUCING TERMINAL DIFFERENTIATION WITH INTERLEUKIN-6

### BACKGROUND

Circulating blood cells are constantly being replaced by newly developed cells. Replacement blood cells are formed in a process termed hematopoiesis in which at least eight mature blood cell types within two major lineages are produced: (1) myeloid, which includes red blood cells (erythrocytes), macrophages (monocytes), eosinophilic granulocytes, megakaryocytes (platelets), neutrophilic granulocytes, basophilic granulocytes (mast cells); and (2) lymphoid, which includes T lymphocytes, and B lymphocytes; Burgess and Nicola, Growth Factors and Stem Cells (Academic Press, New York, 1983). Much of the control of blood cell growth and differentiation is mediated by a group of interacting glycoproteins termed colony stimulating factors (CSFs). These glycoproteins are so named because of the in vivo and in vitro assays used to detect their presence. Techniques for the clonal culture of hematopoietic cells in semisolid culture medium have been especially important in the development of in vitro assays. In such cultures, individual progenitor cells (i.e., cells developmentally committed to a particular lineage, but still capable of proliferation) are able to proliferate to form a colony of maturing progeny in a manner which is believed to be essentially identical to the comparable process in vivo. The role of CSFs in hematopoiesis is the subject of many recent reviews, e.g. Metcalf, The Hemopoietic Colony Stimulating Factors (Elsevier, New York, 1984); Metcalf, Science, Vol. 229, pgs. 16-22 (1985); Nicola et al., Immunology Today, Vol. 5, pgs. 76-80 (1984); Whetton et al., TIBS, Vol. 11, pgs. 207-211 (1986); Clark and Kamen, Science, Vol. 236, pgs. 1229-1237 (1987); and Sachs, Science, Vol. 238, pgs. 1374-1379 (1987).

CSFs are also believed to play a role in the development and progression of myeloid leukemias. Myeloid leukemias are clonal neoplasms of granulocyte-macrophage precursor cells and fall into two major groups: chronic myeloid leukemia (CML) and acute myeloid leukemia (AML).

CML is characterized by expansion in the marrow of the granulocyte-monocyte population at all stages of maturation with massive enlargement of hematopoietic populations in the spleen and blood. Although chemotherapy is successful in reducing the excessive size of the leukemic cell populations, conventional regimens have not succeeded in preventing terminal acute transformation (of progressively higher proportions of cells into immature or abnormal forms) or in extending the life spans of afflicted patients (Metcalf, cited above, 1984).

AML is characterized by an accumulation of immature granulocyte-monocyte blast cells with often little or no evidence of maturing granulocyte-monocyte cells. The disease primarily involves the bone marrow, and spleen enlargement usually is only moderate. Total blood nucleated cells may or may not be elevated, but there is a high proportion of immature blast cells associated with relatively few mature cells. There is usually an associated anemia and thrombocytopenia, and a relative absence in the marrow and peripheral blood of mature granulocytes and monocytes. Death usually results from uncontrollable hemorrhage or overwhelming infections (Metcalf, cited above, 1984).

An important approach to treating myeloid leukemias has involved the identification of agents which are capable of inducing the immature leukemic cells to differentiate into functional mature cells, e.g. Hozumi, Adv. Cancer Res., Vol. 38, pgs. 121-169 (1983). A large number of such agents having varying efficiencies over a spectrum of leukemic cells have been identified by in vitro assays. They include G-CSF (e.g. Tomida et al., FEBS Letters, Vol. 207, pgs. 271-275 (1986)), lipopolysaccharide, glucocorticoid hormones, and certain vitamins (Hozumi, cited above). Some clinical success has been achieved using these agents; however, no agent tested so far appears to have effective differentiation-inducing activity over a broad range of leukemic cell types. It would be valuable to the medical community if additional agents were available, either with broader ranges of activity than those of currently available agents, or with differentiation-inducing activities complementary to those of currently available agents.

### SUMMARY OF THE INVENTION

The invention is a method of treating myeloid leukemias by inducing differentiation with interleukin-6 (IL-6). The invention is based on the discovery that IL-6, also known as B-cell growth factor-2 and interferon-$\beta_2$ (see Sehgal et al., Science, Vol. 235, pgs. 731-732 (1987)), causes myeloid leukemia cells to terminally differentiate into mature cell types in a dose-dependent manner.

### Detailed Description of the Invention

The invention involves administering an effective amount of IL-6 to a patient suffering from myeloid leukemia. As used herein "effective amount" means an amount sufficient to beneficially reduce the proportion of immature leukemic cells in a patient's peripheral blood. The effective amount for a particular patient may vary depending on such factors as the state of the leukemia being treated, the overall health of the patient, the method of administration, the severity of side-effects, and the like.

Generally, IL-6 is administered as a pharmaceutical composition comprising an effective amount of IL-6 and a pharmaceutical carrier. A pharmaceutical carrier can be any compatible, non-toxic substance suitable for delivering the compositions of the invention to a patient. Generally, compositions useful for parenteral administration of such drugs are well known; e.g. Remington's Pharmaceutical Science, 15th Ed. (Mack Publishing Company, Easton, PA 1980). Alternatively, compositions of the invention may be introduced into a patient's body by an implantable drug delivery system; e.g. Urquhart et al., Ann. Rev. Pharmacol. Toxicol., Vol. 24, pgs. 199-236 (1984).

When administered parenterally, the IL-6 is formulated in a unit dosage injectable form (typically a solution, suspension or emulsion) in association with a pharmaceutical carrier. Such carriers are inherently non-toxic and nontherapeutic. Examples of such carriers are normal saline, Ringer's solution, dextrose solution, and Hank's solution. Nonaqueous carriers such as fixed oils and ethyl oleate may also be used. A preferred carrier is 5% dextrose/saline. The carrier may contain minor amounts of additives such as substances that enhance isotonicity and chemical stability, e.g., buffers and preservatives. The IL-6 is preferably formulated in purified form substantially free of aggregates and other proteins at a concentration in the range of about 5 to 20 µg/ml.

Preferably, IL-6 is administered by continuous infusion so that an amount in the range of about 50-800 µg is delivered per day (i.e. about 1-16 µg/kg/day). The daily infusion rate may be varied based on monitoring of side effects and blood cell counts.

IL-6 for use with the invention is prepared by expression of an IL-6 gene in an expression host as described by (e.g.): Asagoe et al., Biotechnology, Vol. 6, pgs. 806-809 (1988) (bacterial expression); Kurjan et al., U.S. patent 4,546,082 (yeast expression); or Clark et al., U.S. patent 4,675,285, or Takabe et al., Mol. Cell. Biol., Vol. 8, pgs. 466-472 (1988) (mammalian expression).

Preferably, IL-6 is purified from culture supernatants of mammalian cells transiently transfected or stably transformed by an expression vector carrying an IL-6 gene. Most preferably, IL-6 is purified from culture supernatants of COS 7 cells transiently transfected by the pcD expression vector pcD-humanIL6 (L1-121), available from the American Type Culture Collection (ATCC) (Rockville, Maryland) under accession number 67748. COS 7 cells are also available from the ATCC under accession number CRL 1651. Transfection of COS 7 cells with pcD-humanIL6 (L1-121) proceeds as follows: One day prior to transfection, approximately $10^6$ COS 7 monkey cells are seeded onto individual 100 mm plates in Dulbecco's modified Eagle medium (DME) containing 10% fetal calf serum and 2 mM glutamine. To perform the transfection, the medium is aspirated from each plate and replaced with 4 ml of DME containing 50 mM Tris.HC1 pH 7.4, 400 µg/ml DEAE-Dextran and 50 µg of plasmid DNA. The plates are incubated for four hours at 37°C, then the DNA-containing medium is removed, and the plates are washed twice with 5 ml of serum-free DME. DME is added back to the plates which are then incubated for an additional 3 hrs at 37°C. The plates are washed once with DME, and then DME containing 4% fetal calf serum, 2 mM glutamine, penicillin and streptomycin is added. The cells are then incubated for 72 hrs at 37°C, after which the growth medium is collected for purification of IL-6.

Plasmid DNA for the transfections can be obtained by growing pcD-humanIL-6 (L1-121) in E. coli MC1061, described by Casadaban and Cohen, J. Mol. Biol., Vol. 138, pgs. 179-207 (1980), or like organism. The plasmid DNA is isolated from the cultures by standard techniques, e.g. Maniatis et al., Molecular Cloning: A Laboratory Manual (Cold Spring Harbon Laboratory, New York, 1982). Alternatively, genes encoding human IL-6 can be purchased commercially from British Biotechnology, Ltd. (Oxford, England). Such genes come in a pUC cloning vector; thus they need to be re-cloned into an expression vector, such as one of those listed above.

Preferably, the IL-6 is separated from the culture supernatants by the following series of steps. First, the supernatants are combined and concentrated, e.g. ten-fold, by ultrafiltration, e.g. using a Millipore (Bedford, MA) YM5 ultrafiltration membrane, or like membrane. The concentrated supernatant is loaded onto a pH-stable C-8 reverse phase high pressure liquid chromatography (HPLC) column, e.g. VYDAC (Hesperia, CA) C-8 coated macroporous silica column, or like column. A human IL-6-containing fraction is eluted from the column by running a gradient of solution $A_1$ (0.10 M sodium borate, pH 9.25) and solution $B_1$ (75% acetonitrile mand 0.02 M sodium borate, pH 9.25); the IL-6 fraction comes off the column with about 50% solution $B_1$. For at least 2-3 liters of concentrated supernatant a 10 mm x 250 mm column can be used. Fractions can be assayed for human IL-6 activity by a variety of biological assays; e.g. Wong et al., Immunology Today, Vol. 9, pgs. 137-139 (1988), list several assays. Fractions can also be assayed by anti-peptide antisera, which are readily produced against the N-terminus of human IL-6, e.g. Hirano et al., Proc. Natl. Acad. Sci., Vol. 84, pgs. 228-231 (1987). Preferably, human IL-6 is assayed by its ability to support the proliferation of the plasmacytoma cell line, MOPC-315, available from the ATCC under accession number TIB 23. The pH of the eluted IL-6 fraction is adjusted to pH 2.0 by adding diluted trifluoroacetic acid (TFA), and the pH-adjusted fraction is loaded onto a second reverse phase HPLC column comprising the same C-8 substrate as the first column. Essentially pure human IL-6 is eluted from the second column by running a gradient of solution $A_2$ (0.1% TFA in water, pH 2.0) and solution $B_2$ (15% isopropyl alcohol, 75% acetonitrile, and 0.1% TFA in water); the IL-6 fraction is removed from the column at about 45% solution $B_2$. For a starting volume of 2-3 liters of concentrated supernatant loaded onto the first column, the dimensions of the second column can be 46 mm by 250 mm.

The descriptions of the foregoing embodiments of the invention have been presented for purpose of illustration and description. They are not intended to be exhaustive or to limit the invention to the precise forms disclosed, and obviously many modifications and variations are possible in light of the above teaching. The embodiments are chosen and described in order to best explain the principles of the invention and its practical application to thereby enable others skilled in the art to best utilize the invention in various embodiments and with various modifications as are suited to the particular use contemplated. It is intended that the scope of the invention be defined by the claims appended hereto.

Applicants have deposited pcD-humanIL6 (L1-121) with the American Type Culture Collection, Rockville, MD, USA (ATCC), under accession number 67748. This deposit is made under conditions as provided under ATCC's agreement for Culture Deposit for Patent Purposes, which assures that the deposit will be made available to the US Commissioner of Patents and Trademarks pursuant to 35 U.S.C. 122 and 37 C.F.R. 1.14, and still be made available to the public upon issue of a U.S. patent, which requires that the deposit be maintained. Availability of the deposited strain is not to be construed as a license to practise the invention in contravention of the rights granted under the authority of any government in accordance with its patent laws.

The Deposit has been modified to conform to the requirements of the Budapest Treaty on the Deposit of Microorganisms.

## Claims

1. A method of treating myeloid leukemias comprising the step of administering an effective amount of human interleukin-6.

2. The method of claim 1 wherein said step of administering includes intravenous delivery of an amount of human interleukin-6 in the range of about 1-16 $\mu$g per kilogram of body weight per day.

3. The method of claim 2 wherein said myeloid leukemia is acute myeloid leukemia.

**DECLARATION**
which under Rule 45 of the European Patent Convention shall be considered, for the purpose of subsequent proceedings, as the European search report

European Patent Office

Application number

EP 89 30 8435

| The Search Division considers that the present European patent application does not comply with the provisions of the European Patent Convention to such an extent that it is not possible to carry out a meaningful search into the state of the art on the basis of all claims.<br>Reason: | CLASSIFICATION OF THE APPLICATION (Int. Cl.4) |
|---|---|
| The pharmacological activity claimed is not supported by examples (EPC Rule 27(1)f, EPC Art. 83,84). The claims were considered to be drafted in the correct way:<br>Use of a compound for the preparation of a medicament, see Official Journal EPO 3 - 1985, pages 64-70 | A 61 K 37/02 |

| Place of search | Date of completion of the search | Examiner |
|---|---|---|
| The Hague | 07-11-1989 | ISERT |

EPO Form 1504 06.78